# DEMANDE DE BREVET EUROPEEN

(11) **EP 1 095 649 A1**
(43) Date de publication de la demande: **02.05.2001**
(21) Numéro de dépôt: 00402407.1
(22) Date de dépôt: 31.08.2000
(51) Int. Cl.: A61K 7/02, C08L 83/04, C08K 5/02, C08K 5/357, A61K 7/48

(54) **Composition anhydre contenant un composé fluoré volatil et ses utilisations notamment cosmétiques**

(30) Priorité: 27.10.1999 FR 9913448
(71) Demandeur: L'OREAL, 75008 Paris (FR)
(72) Inventeur: Lorant, Raluca, 94320 Thiais (FR)
(74) Mandataire: Rasson, Catherine

(57) **Abrégé**

La présente demande concerne une composition anhydre comprenant dans une phase grasse, au moins un organopolysiloxane solide élastomère au moins partiellement réticulé et au moins une huile fluorée volatile de densité supérieure à 1.

L'invention concerne également l'utilisation de la composition selon l'invention pour le traitement, la protection, le soin, le démaquillage et/ou le nettoyage de la peau, des lèvres et/ou des cheveux, et/ou pour le maquillage de la peau et/ou des lèvres.

## Description

La présente demande concerne une composition anhydre comprenant dans une phase grasse, au moins un organopolysiloxane solide élastomère réticulé et au moins une huile fluorée volatile de densité supérieure à 1, et à l'utilisation de ladite composition notamment dans le domaine cosmétique pour le traitement, la protection, le soin, le démaquillage et/ou le nettoyage de la peau, des lèvres et/ou des cheveux, et/ou pour le maquillage de la peau et/ou des lèvres.

Les compositions anhydres constituent d'excellents supports cosmétiques ou dermatologiques notamment en tant que véhicules d'actifs. Ces compositions permettent aussi de former à la surface de la peau un film lipidique qui prévient la perte d'eau transépidermique et protège la peau des agressions extérieures Toutefois, ces compositions présentent l'inconvénient de manquer de confort et d'agrément cosmétique car, de part l'absence de l'eau, elles manquent de fraîcheur et elles paraissent particulièrement lourdes et inconfortables même lorsqu'elles ne contiennent pas d'huiles nourrissantes connues pour apporter un effet gras.

Pour remédier à cet inconvénient, il est connu d'incorporer dans des compositions anhydres, des gels à base d'organosiloxane élastomère réticulé, comme ceux décrits dans le document US-A-4,987,168, ces gels améliorant les qualités cosmétiques de ces compositions notamment en apportant de la douceur, de la matité et un toucher moins gras.

Toutefois, les compositions obtenues, si elles paraissent moins grasses, manquent de fraîcheur lors de l'application sur la peau.

La demanderesse a découvert de façon inattendue que l'utilisation d'une huile fluorée volatile de densité supérieure à 1, permettait de réaliser des compositions anhydres ayant de très bonnes propriétés cosmétiques et apportant un effet de fraîcheur et de confort lors de l'application sur le peau, que l'on n'obtient pas avec les compositions de l'art antérieur.

Ainsi, la présente invention se rapporte à une composition anhydre comprenant, dans une phase grasse, au moins un organopolysiloxane solide élastomère au moins partiellement réticulé, et au moins une huile fluorée volatile de densité supérieure à 1.

On entend par « phase grasse » une phase comprenant des corps gras et notamment des huiles.

Selon l'invention, la densité de l'huile fluorée volatile est généralement supérieure à environ 1,1 et de préférence supérieure à 1,2.

Par l'expression "huile fluorée volatile", on doit entendre une huile ayant, à 25°C, une pression de vapeur saturante au moins égale à 50 Pa.

Parmi les huiles fluorées volatiles répondant aux critères ci-dessus de densité et de pression de vapeur, on peut notamment citer les composés fluorés suivants :
1°) Les perfluorocycloalkyles répondant à la formule (I) suivante : 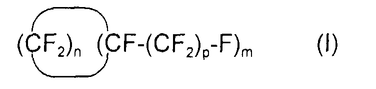 dans laquelle :
   n est 4 ou 5,
   m est ou 2, et
   p est 1, 2 ou 3,
   sous réserve que lorsque m = 2, les groupements ne sont pas nécessairement en alpha, l'un par rapport à l'autre ;
2°) Les perfluoroalcanes répondant à la formule (II) suivante :

   CF₃-(CF₂)ₘ-CF₂X (II)

   dans laquelle :
   m est 2 à 8, et
   X représente Br ou F ;
3°) Les fluoroalkyles ou hétérofluoroalkyles répondant à la formule (III) suivante :

   CH₃-(CH₂)ₙ-[Z]ₜ-(CF₂)ₘ-CF₃ (III)

   dans laquelle :
   test 0 ou 1,
   n est 0, 1, 2 ou 3,
   m est 2, 3, 4 ou 5, et
   Z est O, S ou NR, R étant un hydrogène, un radical -(CH₂)_{P}-CH₃, ou -(CF₂)_{P}-CF₃, p étant 2, 3, 4 ou 5, et
4°) les dérivés de perfluoromorpholine répondant à la formule (IV) suivante : 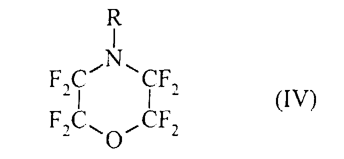 dans laquelle R est un radical perfluoroalkyle en C₁-C₄.

Parmi les perfluorocycloalkyles de formule (I), on peut notamment citer le perfluorométhylcyclopentane et le perfluoro-1,3-diméthylcyclohexane vendus sous les dénominations de "Flutec PC1®" et "Flutec PC 3®" par la Société BNFL Fluorochemicals Ltd., qui ont des densités respectives de 1,26 et 1,82, ainsi que le perfluoro-1,2-diméthylcyclobutane.

Parmi les perfluoroalcanes de formule (II), on peut citer, entre autres, le dodécafluoropentane et le tetradécafluorohexane, vendus sous les dénominations de "PF 5050®" et "PF 5060®" par la Société 3M et qui ont des densités respectives de 1,63 et 1,68 ou encore le bromoperfluorooctyle vendu sous la dénomination de "FORALKYL®" par la Société Atochem, de densité 1,95.

Parmi les composés fluorés de formule (III), on peut citer par exemple, le nonafluorométhoxybutane vendu sous la dénomination de "MSX 4518®" par la Société 3M, de densité 1,53, et le nonafluoroéthoxyisobutane de densité 1,43.

Enfin, parmi les dérivés de perfluoromorpholine, on peut citer par exemple la 4-trifluorométhyl perfluoromorpholine vendue par la Société 3M sous la dénomination "PF 5052®", de densité égale à 1,7.

Les composés fluorés tels que décrits ci-dessus sont par ailleurs caractérisés par un point d'ébullition généralement compris entre 25 et 65°C.

La proportion en huile fluorée volatile dans la composition de l'invention peut varier dans une large mesure et va généralement de 0,5 à 60 % et de préférence de 5 à 50 % en poids par rapport au poids total de la composition.

La composition de l'invention contient au moins un organopolysiloxane solide élastomère au moins partiellement réticulé. On entend par « élastomère » un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomères utilisés dans la composition selon l'invention sont partiellement ou totalement réticulés. Inclus dans une phase huileuse, ils se transforment, selon le taux de phase huileuse utilisé, d'un produit d'aspect spongieux lorsqu'ils sont utilisés en présence de faibles teneurs en phase huileuse en un gel homogène en présence de quantités de phase huileuse plus élevées. La gélification de la phase huileuse par ces élastomères peut être totale ou partielle.

Les élastomères de l'invention peuvent être véhiculés sous forme de gel constitué d'un organopolysiloxane élastomère, incluant au moins une huile hydrocarbonée et/ou une huile de silicone et/ou une huile fluorée. Aussi, la phase huileuse associée à l'organopolysiloxane élastomère peut être constituée de cette ou ces huiles.

Les organopolysiloxanes élastomères utilisés selon l'invention peuvent être choisis parmi les polymères réticulés décrits dans la demande EP-A-0295886.

Selon cette demande, ils sont obtenus par réaction d'addition et de réticulation, en présence d'un catalyseur du type platine, d'au moins :
- (a) un organopolysiloxane ayant au moins deux groupes alcényle inférieurs par molécule, ces groupes alcényle comportant deux à six atomes de carbone ; et
- (b) un organopolysiloxane ayant au moins deux atomes d'hydrogène liés à un atome de silicium par molécule.

Les organopolysiloxanes élastomères utilisés dans la composition de l'invention peuvent aussi être choisis parmi ceux décrits dans le brevet US-A-5,266,321. Selon ce brevet, ils sont choisis notamment parmi :
- i) les organopolysiloxanes comprenant des motifs R₂SiO et RSiO_{1,5} et éventuellement des motifs R₃SiO_{0,5} et/ou SiO₂, dans lesquels les radicaux R, indépendamment les uns des autres, représentent un hydrogène, un radical alkyle tel que méthyle, éthyle ou propyle, un radical aryle tel que phényle ou tolyle, un groupe aliphatique insaturé tel que vinyle, le rapport en poids des motifs R₂SiO sur les motifs RSiO_{1,5} allant de 1/1 à 30/1 ;
- ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 moles % lorsque l'organopolysiloxane est non cyclique et entre 1 et 50 moles % lorsque l'organopolysiloxane est cyclique.

Les organopolysiloxanes de la composition de l'invention sont par exemple ceux commercialisés sous les noms KSG 6 de Shin-Etsu, Trefil E-505C ou Trefil E-506C de Dow-Corning, Gransil (SR-CYC, SR DMF10, SR-DC556) de Grant Industries, ou ceux commercialisés sous forme de gels déjà constitués : KSG 15, KSG 17, KSG 16, KSG 18, KSG 26A, KSG 26B, de Shin-Etsu, Gransil SR 5CYC gel, Gransil SR DMF 10 gel, Gransil SR DC556 gel de Grant Industries, 1229-02-167 et 1229-02-168 de General Electric. On peut aussi utiliser un mélange de ces produits commerciaux.

De façon préférentielle, le ou les organopolysiloxanes élastomères utilisés selon l'invention sont présents en une concentration en matière active allant de 0,1 à 20 %, de préférence de 0,5 à 15 % et mieux de 1,5 à 15 % du poids total de la composition.

La phase huileuse utilisée lors de la fabrication du gel anhydre d'organopolysiloxane élastomère contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les polydiméthylsiloxanes à chaîne linéaire ou cyclique, liquides à température ambiante comportant éventuellement une chaîne alkyle ou aryle pendante ou en bout de chaîne, la chaîne alkyle ayant de 1 à 6 atomes de carbone.

Outre les huiles éventuellement présentes dans le gel d'organopolysiloxane élastomère, la phase grasse de la composition selon l'invention peut être de toute nature et comprendre des huiles, des cires ou des gommes solides à température ambiante, des corps gras pâteux, d'origine animale, végétale, minérale ou synthétique et leurs mélanges.

Comme huiles utilisables dans la composition de l'invention, on peut citer notamment :
- les huiles hydrocarbonées d'origine animale, telles que le perhydrosqualène ;
- les huiles hydrocarbonées d'origine végétale, telles que les triglycérides liquides d'acides gras, par exemple les huiles de tournesol, de maïs, de soja, de courge, de pépins de raisin, de sésame, de noisette, de noyaux d'abricot, de macadamia, de ricin, d'avocat, la fraction liquide du beurre de karité, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin, le myristate d'isopropyle, des octanoates, décanoates ou ricinoléates d'alcools ou de polyalcools ;
- les hydrocarbures linéaires ou ramifiés, d'origine minérale ou synthétique, tels que les huiles de paraffine volatiles ou non et leurs dérivés, comme l'isoparaffine hydrogéné, la vaseline, les polydécènes, le polyisobutène hydrogéné tel que le parléam ;
- les éthers de synthèse de formule R³COR⁴ dans laquelle R³ est un radical alkyle en C₃ à C₁₉ et R⁴ un radical alkyle en C₃ à C₂₀ ;
- des alcools gras comme l'octyldodécanol ou l'alcool oléique ;
- les huiles fluorées partiellement hydrocarbonées et/ou siliconées comme les perfluoropolyesters ;
- les huiles de silicone comme les polyméthylsiloxanes à chaîne siliconée linéaire ou cyclique, liquides ou pâteux à température ambiante, tels que les cyclométhicones (notamment cyclohexaméthicone et cyclopentaméthicone), les phényldiméthicones, les phényltriméthicones, les polyméthylphénylsiloxanes, les alkylpolydiméthyl-siloxanes avec une chaîne alkyle en C₂ à C₂₀ ;
- leurs mélanges.

La composition selon l'invention peut être appropriée notamment pour une utilisation topique et constituer en particulier une composition cosmétique et/ou dermatologique. Elle contient alors un milieu physiologiquement acceptable. On entend par "physiologiquement acceptable" un milieu compatible avec la peau, les yeux et les fibres kératiniques (cheveux, cils) des êtres humains.

De façon connue, les compositions de l'invention peuvent contenir des adjuvants habituels dans les domaines considérés, tels que des actifs, des gélifiants lipophiles, des conservateurs, des antioxydants, des parfums, des solvants, des charges, des filtres, des matières colorantes, des agents basiques ou acides et encore des vésicules lipidiques. Ces adjuvants sont utilisés dans les proportions habituelles dans les domaines considérés, et par exemple de 0,01 à 20 % du poids total de la composition. Ces adjuvants ainsi que leurs concentrations doivent être tels qu'ils ne modifient pas la propriété recherchée pour la composition de l'invention.

Comme gélifiants lipophiles, on peut citer les argiles modifiées comme les bentones, les sels métalliques d'acides gras, la silice hydrophobe et les polyéthylènes.

Comme actifs, on peut citer notamment les actifs lipophiles tels que les filtres solaires liposolubles comme l'octylmethoxycinnamate ; les vitamines et notamment les vitamines A, E, F et D et leurs dérivés comme le palmitate de vitamine A, le 7-dehydrocholestérol ou provitamine D3, l'acétate de tocophérol ; les acides gras insaturés comme l'acide linoléique et l'acide linolénique ; l'alphabisabolol ; les beurres d'origine végétale citées ci-dessus parmi les huiles, comme le beurre de Shorea ou le beurre de karité qui reconstituent la barrière lipidique de la peau et permettent le traitement des peaux sèches.

Par ailleurs, la composition de l'invention a l'avantage de permettre de stabiliser tout actif notamment hydrophile, instable en milieu oxydant, et on peut citer notamment comme actifs instables en milieu oxydant, les vitamines et notamment l'acide ascorbique (vitamine C) et ses dérivés, notamment ses dérivés glycosylés et phosphatés, et ses esters comme l'acétate, le palmitate et le propionate d'ascorbyle, le rétinol (vitamine A) et ses dérivés, notamment ses esters comme l'acétate et le propionate de rétinol ; l'urée ; la rutine ; les enzymes telles que la lipase, la protéase, la phospholipase et les cellulases ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, l'acide rétinoique et ses dérivés, l'acide benzène 1,4-di-(3-méthylidène 10-camphosulfonique) ; les caroténoïdes tels que les carotènes comme par exemple les α-, β- et γ-carotènes, le β,ϕ-carotène, le ξ-carotène, le β,λ-carotène, le lycopène (ψ,ψ-carotène) ; les acides gras polyinsaturés tels que l'acide gamma-linolénique, et leurs mélanges.

Il peut s'agir également de tous les composés naturels ou synthétiques pouvant contenir les actifs indiqués ci-dessus, en particulier les extraits végétaux, et plus spécialement les extraits de fruits.

Comme charges susceptibles d'être utilisées dans la composition de l'invention, on peut citer par exemple le talc ; les particules de polyamide et notamment celles vendues sous la dénomination Orgasol par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de Polytrap ; les poudres expansées telles que les microsphères creuses et notamment, les microsphères commercialisées sous la dénomination commerciale Expancel par la société Kemanord Plast ou sous la dénomination commerciale Micropearl F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination Dry-Flo par la société National Starch ; les microbilles de résine de silicone telles que celles commercialisées sous la dénomination Tospearl par la société Toshiba Silicone ; et leurs mélanges.

La composition, objet de l'invention, trouve son application notamment dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres. Elle peut être destinée aussi au traitement des peaux sèches et/ou des lèvres sèches.

La composition selon l'invention peut, par exemple, être utilisée comme produit de soin, de démaquillage et/ou de nettoyage pour le visage sous forme de crème ou de lait, comme produit de maquillage (peau et lèvres) par incorporation de charges ou de colorants ou comme produits solaires par incorporation de filtres.

Aussi, l'invention a encore pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau et/ou des lèvres.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

L'invention a aussi pour objet l'utilisation de la composition telle que définie ci-dessus pour la fabrication d'une composition destinée au soin des peaux sèches et/ou des lèvres sèches.

Les exemples qui suivent permettront de mieux comprendre l'invention, sans toutefois présenter un caractère limitatif. Les quantités indiquées sont en % en poids, sauf mention contraire.

### Exemple 1 : Baume réparateur pour les lèvres

| *Phase gélifiée :* | |
|---|---|
| KSG 6 (Dimethicone / Vinyl dimethicone crosspolymer | 50 % |
| à 60 % de matière active dans dimethicone) | |
| Isoparaffine hydrogéné | 43 % |
| Fraction liquide de beurre de karité | 2 % |

| *Phase fluorée :* | |
|---|---|
| Nonafluoromethoxybutane (MSX 4518 de la société 3M) | 5 % |

Mode opératoire : On disperse la phase fluorée dans la phase gélifiée. On obtient un gel particulièrement doux et agréable à l'application qui lisse immédiatement les lèvres.

### Exemple 2 : Crème poudrée adoucissante

| *Phase gélifiée* | |
|---|---|
| KSG 16 (Dimethicone / Vinyl dimethicone crosspolymer | |
| à 24 % de matière active dans dimethicone) | 40 % |
| Huile de noyaux d'abricot | 10 % |
| Cyclohexamethicone | qsp 100 % |

| *Charges* | |
|---|---|
| Poudre de nylon-12 (Orgasol 2002 de la société Atochem) | 5 % |
| Talc | 16 % |

| *Phase fluorée* | |
|---|---|
| Tetradecafluorohexane (PF 5060 de la société 3M) | 20 % |

Mode opératoire : On disperse les charges dans la phase gélifiée, puis on disperse la phase fluorée dans le mélange obtenu.

On obtient une crème onctueuse poudrée très douce et légère à l'application.

## Revendications

1. Composition anhydre comprenant, dans une phase grasse, au moins un organopolysiloxane solide élastomère au moins partiellement réticulé, et au moins une huile fluorée volatile de densité supérieure à 1.

2. Composition selon la revendication 1, caractérisée par le fait que l'huile fluorée volatile a une densité supérieure à 1,2.

3. Composition selon la revendication 1 ou 2, caractérisée par le fait que l'huile fluorée volatile est un perfluorocycloalkyle répondant à la formule (I) suivante : 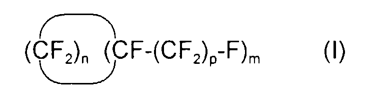 dans laquelle :
n est 4 ou 5,
m est 1 ou 2 et
p est 1, 2 ou 3 ,
sous réserve que lorsque m = 2, les groupements ne sont pas nécessairement en alpha l'un par rapport à l'autre.

4. Composition selon la revendication 1 ou 2, caractérisée par le fait que l'huile fluorée volatile est un perfluoroalcane répondant à la formule (II) suivante :
CF₃-(CF₂)ₘ-CF₂X (II)
dans laquelle :
m est 2 à 8 et
X représente Br ou F.

5. Composition selon la revendication 1 ou 2, caractérisée par le fait que l'huile fluorée volatile est un fluoroalkyle ou hétérofluoroalkyle répondant à la formule (III) suivante :
CH₃-(CH₂)ₙ-[Z]ₜ-(CF₂)ₘ-CF₃ (III)
dans laquelle :
t est 0 ou 1,
n est 0, 1, 2 ou 3,
m est 2, 3, 4 ou 5, et
Z est O, S ou NR, R étant un hydrogène, un radical -(CH₂)_{P}-CH₃, ou -(CF₂)_{P}-CF₃, p étant 2, 3, 4 ou 5.

6. Composition selon la revendication 1 ou 2, caractérisée par le fait que l'huile fluorée volatile est un dérivé de perfluoromorpholine répondant à la formule (IV) suivante : 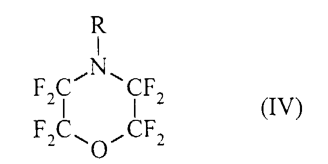 dans laquelle R est un radical perfluoroalkyle en C₁-C₄.

7. Composition selon l'une quelconque des revendications précédentes, caractérisée par le fait que l'huile fluorée volatile est choisie dans le groupe constitué par le perfluorométhylcyclopentane, le perfluoro-1,3 diméthylcyclohexane, le dodécafluoropentane, le tétradécafluorohexane, le bromoperfluorooctyle, le nonafluorométhoxybutane le nonafluoroéthoxyisobutane et la 4-trifluorométhyl perfluoromorpholine.

8. Composition selon l'une quelconque des revendications précédentes, caractérisée par le fait que la proportion en huile fluorée volatile va de 0,5 à 60 % en poids par rapport au poids total de ladite composition.

9. Composition selon l'une quelconque des revendications précédentes, caractérisée par le fait que l'organopolysiloxane élastomère est obtenu par réaction d'addition et de réticulation, en présence d'un catalyseur, d'au moins :
(a) un organopolysiloxane ayant au moins deux groupes alcényle inférieurs par molécule ; et
(b) un organopolysiloxane ayant au moins deux atomes d'hydrogène liés à un atome de silicium par molécule.

10. Composition selon l'une quelconque des revendications précédentes, caractérisée par le fait que l'organopolysiloxane est choisi parmi :
- i) les organopolysiloxanes comprenant des motifs R₂SiO et RSiO_{1,5} et éventuellement des motifs R₃SiO_{0,5} et/ou SiO₂, dans lesquels les radicaux R, indépendamment les uns des autres, représentent un hydrogène, un radical alkyle, un radical aryle, un groupe aliphatique insaturé, le rapport en poids des motifs R₂SiO sur les motifs RSiO_{1,5} allant de 1/1 à 30/1 ;
- ii) les organopolysiloxanes insolubles dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 moles % lorsque l'organopolysiloxane est non-cyclique et entre 1 et 50 moles % lorsque l'organopolysiloxane est cyclique.

11. Composition selon l'une quelconque des revendications précédentes, caractérisée par le fait que l'organopolysiloxane solide élastomère est présent en une concentration en matière active allant de 0,1 à 20 % en poids par rapport au poids total de la composition.

12. Composition selon l'une des revendications précédentes, caractérisée par le fait que la phase grasse contient des huiles, des cires, des gommes, des corps gras pâteux et leurs mélanges.

13. Composition selon l'une quelconque des revendications précédentes, caractérisée par le fait qu'elle constitue une composition cosmétique.

14. Utilisation cosmétique de la composition selon l'une quelconque des revendications 1 à 13, pour le traitement, la protection, le soin, le démaquillage et/ou le nettoyage de la peau, des lèvres et/ou des cheveux, et/ou pour le maquillage de la peau et/ou des lèvres.

15. Procédé de traitement cosmétique de la peau, des cheveux, et/ou des lèvres, caractérisé par le fait qu'on applique sur la peau, les cheveux et/ou les lèvres, une composition selon l'une quelconque des revendications 1 à 13.

16. Utilisation de la composition selon l'une quelconque des revendications 1 à 13, pour la fabrication d'une composition destinée au soin des peaux sèches et/ou des lèvres sèches.
